# EUROPEAN PATENT SPECIFICATION

(11) **EP 2 720 668 B1**
(45) Date of publication and mention of the grant of the patent: **06.05.2020**
(21) Application number: 12729058.3
(22) Date of filing: 05.06.2012
(51) Int. Cl.: A61Q 15/00, C08G 18/48, C08G 18/75, C08G 18/08, C08G 18/32, A61K 8/81, A61K 8/87, C08F 20/52

(54) **COSMETIC USE OF A FLOCCULANT POLYMER AS ANTIPERSPIRANT**
KOSMETISCHE VERWENDUNG EINES FLOCKUNGSPOLYMERS ALS SCHWEISSHEMMENDES MITTEL
UTILISATION COSMÉTIQUE D'UN POLYMÈRE FLOCULANT COMME ANTI-TRANSPIRANT

(30) Priority: 20.06.2011 FR 1155366; 20.06.2011 FR 1155364; 20.06.2011 FR 1155369; 29.06.2011 US 201161502670 P; 29.06.2011 US 201161502652 P; 29.06.2011 US 201161502659 P
(43) Date of publication of application: 23.04.2014
(73) Proprietor: L'Oréal, 75008 Paris (FR)
(72) Inventor: MALLE, Gérard, F-77580 Villiers S/morin (FR); JEGOU, Gwenaëlle, F-91240 Saint Michel sur Orge (FR)
(74) Representative: L'Oreal
(86) International application number: PCT/EP2012/060575
(87) International publication number: WO 2012/175330

(56) References cited:
- WO-A1-95/01383
- WO-A1-2010/070140
- WO-A2-2011/026577
- DE-A1- 10 322 152
- DE-A1-102004 012 363
- FR-A1- 2 871 376
- FR-A1- 2 952 535
- JP-A- 9 118 705
- US-A- 5 869 600

## Description

The present invention relates to the cosmetic use of a flocculant polymer comprising as side chain non-quaternized pyridine groups directly linked or indirectly to the main chain, as antiperspirant active agent, particularly in a composition not containing any antiperspirant aluminium and/or zirconium salts.

A subject of the present invention is also a cosmetic process for treating perspiration, which consists in applying to the surface of the skin a composition comprising, in a cosmetically acceptable medium, at least one flocculant polymer as defined previously, not containing any antiperspirant aluminium and/or zirconium salts.

In the cosmetic field, it is well known to use, in topical application, antiperspirant products containing substances that have the effect of limiting or even preventing the flow of sweat. These products are generally available in the form of roll-ons, sticks, aerosols or sprays.

Antiperspirant substances generally consist of aluminium salts or aluminium/zirconium salts. Their antiperspirant efficacy is limited when they are used alone. The use of these active agents at high concentrations in order to obtain good efficacy leads to formulation difficulties. Furthermore, these substances have a skin irritant potential.

There is thus a need to find novel antiperspirant active agents that can replace aluminium salts and aluminium/zirconium salts, and that are efficient and easy to formulate.

Patent application WO 95/24105 proposes as antiperspirant active agents water-insoluble polymers that form an occlusive film on the skin. The use of standard aluminium salts is unnecessary. The occlusive polymers proposed are of the octylacrylamide/acrylate copolymer or vinyl acetate/butyl maleate/isobornyl acrylate type, alone or in combination with a PVP/linear α-olefin polymer such as PVP/eicosene.

Antiperspirant active agents also proposed in patent application WO 95/27473 are water-insoluble film-forming polymers whose main chain is hydrocarbon-based and which comprise pendent hydrophobic quaternary ammonium groups.

Patent application WO 01/54658 discloses anhydrous compositions containing a cyanoacrylate monomer that reacts with sweat to form *in situ* by polymerization a film on the skin that blocks the sweat ducts.

However, these occlusive film-forming polymers do not make it possible to obtain entirely satisfactory antiperspirant efficacy, and still give rise to formulation problems.

Moisture-absorbing polymers have been proposed as substitutes for standard astringent salts in antiperspirant compositions in patent US 4 743 440. These moisture-absorbing polymers may especially be water-soluble and chosen especially from natural gums (xanthan, agar, carrageenans, guar, gelatin), celluloses (hydroxypropylmethylcellulose, carboxymethylcellulose), polyoxyethylenes, polyvinylpyrrolidones, carboxyvinyl polymers or vinyl ether/maleic anhydride copolymers. In patent application WO 03/030 853, the recommended moisture-absorbing polymers are chosen from grafted starch homopolymers and 2-propenamide-co-propenoic acid sodium salt copolymers.

However, these moisture-absorbing polymers do not make it possible to obtain entirely satisfactory antiperspirant efficacy, and still give rise to formulation problems.

Water-soluble quaternary polymers have been proposed in antiperspirant compositions in the presence of standard aluminium salts in order to improve their efficacy. This is the case for dimethyldiallylammonium chloride in patent application EP 222 580, which acts as an agent for retaining the antiperspirant salt. This is the case for the water-soluble polymers comprising a Bronsted acid in patent application WO 02/49590, in particular those derived from maleic acid and/or maleic anhydride, which act as co-gelling agent with the antiperspirant salts. This is the case for polyethyleneimines (PEI) in the article Cosmetics & Toiletries Vol. 108, August 1993, pp. 73-77, which act as agents for complexing the aluminium salts.

Dimethyldiallylammonium chloride/acrylic acid copolymers were proposed in patent application EP 478 327 as thickeners in aqueous liquid antiperspirant products containing aluminium salts.

In patent US 4 690 817, film-forming polymers of vinyl alcohols containing quaternary amine side groups were proposed in antiperspirant compositions in the presence of standard astringent salts, as skin-conditioning agents that form a moisturizing barrier thereon.

In patent application WO 82/01993, polyethyleneimines were used as odour absorbers, in particular for fatty acids, aldehydes or ketones, and more particularly in alcohol-based deodorant products in spray or roll-on form.

US5869600 discloses a method of controlling perspiration which comprises applying to an area of the skin a composition comprising a specific film-forming polymer dissolved in a cosmetically acceptable non-aqueous solvent carrier, said film-forming polymer possibly comprising 2-pyridyl or 4-pyridyl side-chains.

The Applicant has discovered, surprisingly, that particular flocculant polymers constitute by themselves excellent antiperspirant agents and can be readily formulated in numerous products intended for treating perspiration and the body odour associated with perspiration, without it being necessary to use standard astringent salts.

The present invention relates to the cosmetic use of a flocculant polymer comprising as side chain non-quaternized pyridine groups directly linked or indirectly to the main chain, as antiperspirant active agent, particularly in a composition not containing any antiperspirant aluminium and/or zirconium salts.

A subject of the present invention is also a cosmetic process for treating perspiration, which consists in applying to the surface of the skin a composition comprising, in a cosmetically acceptable medium, at least one flocculant polymer as defined previously, not containing any antiperspirant aluminium and/or zirconium salts.

The term "antiperspirant agent" means any substance which, by itself, has the effect of reducing or limiting the flow of sweat without it being necessary to use an aluminium and/or zirconium antiperspirant salt.

The term "effective amount of polymer" means an amount of polymer that is sufficient to be able to observe after application to the surface of the skin an antiperspirant effect as defined above.

The term "cosmetically acceptable medium" means a medium that is compatible with the skin and/or its integuments or mucous membranes, having a pleasant colour, odour and feel and not causing any unacceptable discomfort (stinging, tautness or redness) liable to discourage the consumer from using this composition.

The term "flocculant polymer" means any polymer that is capable of destabilizing a colloidal suspension indiscriminantly via a flocculation or coagulation mechanism. The term "destabilizing colloids" means the formation of aggregates that make the suspension unstable. As the terms "flocculation" and "coagulation" are generally interchangeable and equivalent, we will speak in the invention of "flocculation" for one or other of the mechanisms. The definitions of these mechanisms are given in volume 10 of Kirk-Othmer's Encyclopaedia of Chemical Technology, 3rd edition.

The term "composition not containing any antiperspirant aluminium and/or zirconium salts" means any composition containing not more than 1% by weight of antiperspirant aluminium and/or zirconium salts.

### Flocculant polymers comprising as side chain non-quaternized pyridine groups directly or indirectly linked to the main chain

The flocculant polymers of the invention may be in the form of homopolymer or copolymer containing at least one monomer of non-quaternized pyridine type.

The flocculant polymers of the invention are preferably water-dispersible or water-soluble.

The term "water-dispersible polymer" means any polymer which, when introduced into an aqueous phase (into water or into a mixture of water and linear or branched C₂-C₅ monoalcohols, for instance ethanol, isopropanol or n-propanol) at room temperature (25°C and 1 atmosphere), without pH modification, at a solids content allows the production of a dispersion whose mean particle size is between 5 nm and 5 µm.

The term "water-soluble polymer" means any polymer which, when introduced into water or into a mixture of water and linear or branched C₂-C₅ monoalcohols, for instance ethanol, isopropanol or n-propanol, without pH modification at 25°C, to a mass concentration equal to 1%, allows the production of a macroscopically homogeneous and transparent solution, i.e. a solution with a minimum light transmission value, at a wavelength equal to 500 nm, through a sample 1 cm thick, of at least 80% and preferably at least 90%.

The molar masses of these polymers generally range from 1000 to 20 000 000 g/mol and preferably between 5000 and 1 000 000 g/mol.

The polymers may be crosslinked or non-crosslinked.

Among the flocculant polymers comprising non-quaternized pyridine groups that are pendent relative to the main chain, which may be used in the compositions of the invention, mention may be made of homopolymers or copolymers comprising at least one monomer (C) to (F) defined below: 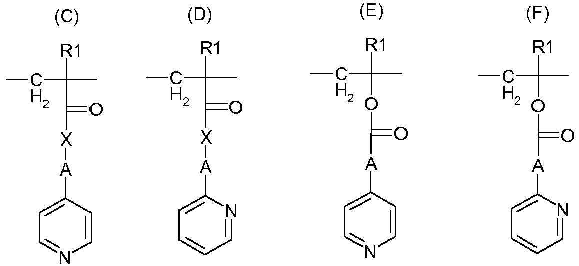 where:
X =O, or NH
R1 represents a hydrogen atom or an alkyl group containing from 1 to 3 carbon atoms, preferably methyl;
A is a linear or branched C₁-C₆ and preferably C₂-C₃ alkyl group; a C₁-C₄ hydroxyalkyl group, preferably monohydroxyalkyl;
The monomers corresponding to the preferred units among (C)-(F) are: N-(4-pyridyl)propylmethacrylamide, N-4(pyridyl)ethyl methacrylate.

According to one particular form of the invention, the flocculant polymer also comprises units consisting of at least one cationic monomer other than a pyridine unit, which may be chosen from quaternary ammonium monomers and (meth)acrylamide monomers, or mixtures thereof.

Among these additional cationic monomers, mention may be made of those having the following formulae: 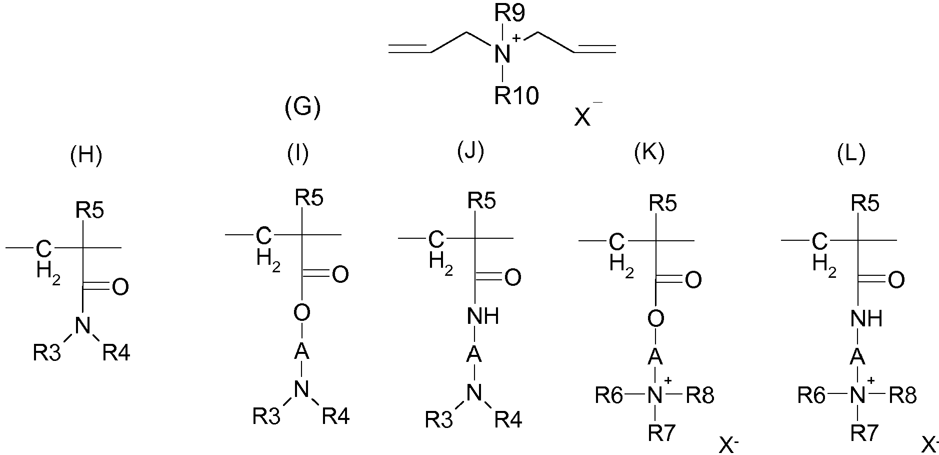 in which:
Rg and R₁₀ independently represent a linear or branched C₁-C₈ alkyl group, a benzyl group, a C₁-C₅ hydroxyalkyl group or a linear or branched amido(C₁-C₄)alkyl group;
R₃ and R₄ independently represent a hydrogen atom or a linear or branched alkyl group having from 1 to 6 carbon atoms, and preferably methyl or ethyl;
R₃ and R₄ may form with the nitrogen atom a 5- to 8-membered non-aromatic nitrogenous heterocycle, for instance pyrrolidine, piperazine or piperidine;
R₅ represents a hydrogen atom or an alkyl group containing from 1 to 3 carbon atoms, preferably methyl;
A is a linear or branched C₁-C₆ and preferably C₂-C₃ alkyl group; a C₁-C₄ hydroxyalkyl group, preferably monohydroxyalkyl;
R₆, R₇ and R₈, which may be identical or different, represent a hydrogen atom or a linear or branched C₁-C₆ alkyl group or a benzyl radical;
X denotes a mineral anion such as a halide (chloride, bromide or iodide) or an organic anion such as a (C₁-C₄) alkyl sulfate (methyl sulfate or ethyl sulfate).

The preferred units of formula (G) are diallyldimethylammonium chloride (DADMAC) and diallyldiethylammonium chloride (DADEAC).

The preferred units of formula (H) are methacrylamide or acrylamide and more particularly acrylamide.

The units (I), (J), (K) or (L) may be chosen from methylaminoethyl methacrylate, benzyl dimethylaminoethylacrylate chloride (DMAEA-BCQ), acryloyloxyethyltrimethylammonium chloride (AETAC); methacryloyloxyethyltrimethylammonium chloride (METAC); methylacrylamidopropyltrimethylammonium chloride (MAPTAC); acrylamidopropyltrimethylammonium chloride (APTAC); acryloyloxyethyltrimethylammonium methosulfate (AETAMS); methacryloyloxyethyltrimethylammonium methosulfate (METAMS); acryloyloxyethyldiethylmethylammonium chloride; methacryloyloxyethyldiethylmethylammonium chloride or the equivalents thereof neutralized with a methyl sulfate, vinylimidazole, piperidine ethylmethacrylate or piperazine ethylmethacrylate.

The most preferential are benzyl dimethylaminoethylacrylate chloride (DMAEA-BCQ), acryloyloxyethyltrimethylammonium chloride (AETAC), methacryloyloxyethyltrimethylammonium methosulfate (METAMS) and methylaminoethyl methacrylate.

According to one particular form of the invention, the flocculant polymer also comprises units consisting of at least one anionic monomer preferably chosen from those having the following formula: 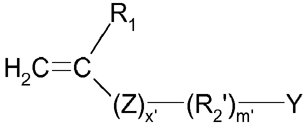 with
Y is a group chosen from -COOH, -SO₃H, -OSO₃H, -PO₃H₂ and -OPO₃H₂. It is understood that, according to the prior art, the groups SO₄H₂ and PO₄H₂ are linked to R'₂ via the oxygen atom, whereas the groups SO₃H and PO₃H are linked to R'₂, respectively, via the atoms S and P.
- R₁ is as defined previously,
- Z is a divalent group chosen from -COO-, -CONH-, -CONCH₃-, -OCO- or -O-, - SO₂- -CO-O-CO- or -CO-CH₂-CO-, preferably Z is chosen from COO and CONH,
- x' = 0 or 1, preferably 1,
- R'₂ is a saturated or unsaturated, optionally aromatic, linear, branched or cyclic carbon-based divalent radical of 1 to 30 carbon atoms, which may comprise 1 to 18 heteroatoms chosen from O, N, S, F, Si and P; the heteroatom(s) may be intercalated in the chain of the said radical R'₂ or alternatively the said radical R'₂ may be substituted with one or more groups comprising them such as hydroxyl or amino.

Among the preferred anionic monomers M when they are present, mention may be made of maleic anhydride, acrylic acid, methacrylic acid, crotonic acid, itaconic acid, fumaric acid, maleic acid, 2-carboxyethyl acrylate (CH₂=CH-C(O)-O-(CH₂)₂-COOH); styrenesulfonic acid, 2-acrylamido-2-methylpropanesulfonic acid, vinylbenzoic acid, vinylphosphoric acid and sulfopropyl (meth)acrylate, and salts thereof.

According to one particular form of the invention, the flocculant polymer also comprises units consisting of at least one nonionic monomer.

The additional nonionic monomers may be chosen especially from those having the following formula, alone or as a mixture: 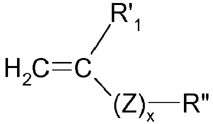 in which:
- R'₁ is hydrogen or -CH₃;
- Z is a divalent group chosen from -COO-, -CONH-, -CONCH₃-, -OCO-,
- SO₂, -CO-O-CO-, -CO-CH₂-CO- and -O-; preferably COO or CONH;
- x is 0 or 1;
- R" is a saturated or unsaturated, optionally aromatic, linear, branched or cyclic carbon-based radical of 1 to 30 carbon atoms, which may comprise 1 to 18 heteroatoms chosen from O, N, S, F, Si and P; the heteroatom(s) may be intercalated in the chain of the said radical or alternatively the said radical R" may be substituted with one or more groups comprising them such as hydroxyl, ester, amide, urethane or urea.

In particular, R" may be a methyl, ethyl, propyl, isopropyl, n-butyl, isobutyl, tert-butyl, phenyl or benzyl radical or a radical of formula -CH₂-CH₂-CH₂OH, -CH₂-CH₂-OH, -CH₂-CH₂-CH₂OH or furfuryl.

The additional nonionic monomers may be chosen especially from the monomers below: methyl methacrylate, methyl acrylate, ethyl methacrylate, ethyl acrylate, propyl acrylate, isopropyl acrylate, tetrahydrofurfuryl methacrylates, butyl methacrylate, 2-ethylhexyl acrylate, stearyl methacrylate, acrolein, tetrahydrofurfuryl acrylate, 2-hydroxyethyl methacrylate, 2-hydroxyethyl acrylate, ethoxyethyl methacrylate, ethoxyethyl acrylate, N-isopropylacrylamide, N-isopropylmethacrylamide, N,N-dimethylacrylamide, N,N-dimethylmethacrylamide, vinyl acetate, methyl vinyl ether, ethyl vinyl ether, vinylpyrrolidone, vinylcaprolactam, N-vinylacetamide, hydroxypropyl acrylate, N-vinyllactam, acrylamide, N-methylacrylamide, N,N-dimethylacrylamide, N-methyl-N-vinylacetamide, N-vinylformamide, N-methyl-N-vinylformamide, vinyl alcohol (copolymerized in the form of vinyl acetate and then hydrolysed).

The polymers containing a non-quaternized pyridine unit may advantageously be totally or partially neutralized relative to the pyridine unit with an organic acid or an inorganic acid:
Neutralization of the amine units, belonging to the functionalized pyridine polymer, may be performed with a mineral acid, such as sulfuric acid, hydrochloric acid, hydrobromic acid, hydriodic acid, phosphoric acid or boric acid; or alternatively with an organic acid, which may comprise one or more carboxylic, sulfonic or phosphonic acid groups. They may be linear, branched or cyclic aliphatic acids, or alternatively unsaturated or aromatic acids. These acids may also include one or more heteroatoms chosen from O, N, Si, F and P, for example in the form of hydroxyl groups. The neutralizers of organic acid type may be chosen from linear, branched or cyclic aliphatic acids and/or unsaturated or aromatic acids, and may especially comprise 1 to 1000 carbon atoms and especially 2 to 500 carbon atoms. They contain at least one Bronsted acid function, and especially one or more carboxylic, sulfonic and/or phosphonic acid groups. They may also include one or more heteroatoms chosen from O, N, Si, F and P, for example in the form of hydroxyl groups.

Linear, branched or cyclic, saturated or unsaturated, optionally aromatic fatty acids containing 6 to 32 carbon atoms, especially 8 to 28, and comprising at least one COOH or sulfonic acid (-SO₃H) function, may be used in particular as neutralizer. Linear, branched or cyclic, saturated or unsaturated, optionally aromatic hydroxy acids and especially α-hydroxy acids containing 2 to 32 and especially 6 to 28 carbon atoms, and comprising at least one COOH or sulfonic acid (-SO₃H) function, may also be used.

Alkylbenzenesulfonic acids in which the alkyl group may comprise from 4 to 30 and especially 6 to 24 carbon atoms may also be used.

Amphoteric neutralizers may also be used, especially of the alkylbetaine or alkylamidopropylbetaine type, in which the alkyl group may comprise 1 to 30, especially 4 to 24 or even 6 to 22 carbon atoms; mention may be made in particular of cocamidopropylbetaine.

Mention may be made especially of α-hydroxyethanoic (or glycolic) acid, α-hydroxyoctanoic acid, α-hydroxycaprylic acid, ascorbic acid, acetic acid, benzoic acid, behenic acid, capric acid, citric acid, caproic acid, caprylic acid, dodecylbenzenesulfonic acid, 2-ethylcaproic acid, folic acid, fumaric acid, galactaric acid, gluconic acid, 2-hexadecyleicosanoic acid, hydroxycaproic acid, 12-hydroxystearic acid, isolauric (or 2-butyloctanoic) acid, isomyristic (or 2-hexyloctanoic) acid, isoarachidic (or 2-octyldodecanoic) acid, isolignoceric (or 2-decyltetradecanoic) acid, lactic acid, lauric acid, malic acid, myristic acid, oleic acid, palmitic acid, propionic acid, sebacic acid, stearic acid, tartaric acid, terephthalic acid, trimesic acid, undecylenic acid, propylbetaine, cocamidopropylbetaine and betaine hydrochloride of formula [(CH₃)₃N⁺CH₂CO₂H.Cl⁻], and also mixtures thereof.

Preferably, caproic acid, 2-ethylcaproic acid, oleic acid, behenic acid, stearic acid, acetic acid, citric acid, tartaric acid, betaine hydrochloride and/or gluconic acid, and preferentially betaine hydrochloride and/or behenic acid, may be used as neutralizer.

Among the polymers of the invention that are particularly preferred, mention may be made of:
- poly(4- or 2-vinylpyridine)-co-styrene copolymers such as those sold by Aldrich;
- poly(4- or 2-vinylpyridine)-co-butyl methacrylate copolymers such as those sold by Aldrich;
- poly(4- or 2-vinylpyridine) crosslinked with divinylbenzene, for instance Poly(4-vinylpyridine), 2% crosslinked with divinylbenzene, sold by Aldrich, and Poly(4-vinylpyridine) ReillexTM 402 crosslinked with divinylbenzene, sold by Reilly Industries, Inc.;
- block copolymers comprising at least one block comprising a pyridine monomer, for instance diblock polymers comprising a polyethylene glycol (PEG) or polyisobutylene block and a poly(4- or 2-vinylpyridine) block;
- neutralized polymers, such as Poly(4-vinylpyridinium p-toluenesulfonate) and Poly(4-vinylpyridine hydrochloride) 2% crosslinked with divinylbenzene, sold by Aldrich.

The polymers according to the invention may preferably be conveyed in aqueous medium, i.e. they are preferably water-soluble or water-dispersible. The dissolution or dispersion in water may be performed by direct dissolution of the polymer if it is soluble, or by neutralization of the amine units so as to make the polymer soluble or dispersible in water. The aqueous dissolution or dispersion may also be performed via an intermediate dissolution step in an organic solvent followed by addition of water before evaporation of the organic solvent.

The polymers according to the invention are preferably conveyed in aqueous medium, i.e. they are water-soluble or water-dispersible. Preferably, the polymers are water-dispersible. The dissolution or dispersion in water may be performed by direct dissolution of the polymer if it is soluble, or by neutralization of the amine units so as to make the polymer soluble or dispersible in water. The aqueous dissolution or dispersion may also be performed via an intermediate dissolution step in an organic solvent followed by addition of water before evaporation of the organic solvent.

The flocculant polymers used as antiperspirant active agents are preferably present in the compositions according to the invention in amounts ranging from 0.1% to 50% by weight and more preferentially from 1% to 20% by weight relative to the total weight of the composition.

### GALENICAL FORMS

The composition according to the invention may be in any galenical form conventionally used for topical application and especially in the form of aqueous gels, or aqueous or aqueous-alcoholic solutions. By adding a fatty or oily phase, it may also be in the form of dispersions of lotion type, emulsions of liquid or semiliquid consistency of milk type, obtained by dispersing a fatty phase in an aqueous phase (O/W) or conversely (W/O), or suspensions or emulsions of soft, semi-solid or solid consistency of the cream or gel type, or alternatively multiple emulsions (W/O/W or O/W/O), microemulsions, vesicular dispersions of ionic and/or nonionic type, or wax/aqueous phase dispersions. These compositions are prepared according to the usual methods.

The compositions may especially be conditioned in pressurized form in an aerosol device or in a pump-action bottle; conditioned in a device equipped with a perforated wall, especially a grille; conditioned in a device equipped with a ball applicator ("roll-on"); conditioned in the form of wands (sticks) or in the form of loose or compacted powder. In this regard, they contain the ingredients generally used in products of this type, which are well known to those skilled in the art.

According to one particular form of the invention, the compositions according to the invention may be anhydrous.

The term "anhydrous composition" means a composition containing less than 2% by weight of water, or even less than 0.5% water, and especially free of water, the water not being added during the preparation of the composition but corresponding to the residual water provided by the mixed ingredients.

According to another particular form of the invention, the compositions according to the invention may be solid, in particular in wand or stick form.

The term "solid composition" means that the maximum force measured by texturometry during the penetration of a probe into the sample of formula must be at least equal to 0.25 newtons, in particular at least equal to 0.30 newtons and especially at least equal to 0.35 newtons, assessed under precise measuring conditions as follows.

The formulae are poured hot into jars 4 cm in diameter and 3 cm deep. Cooling is performed at room temperature. The hardness of the formulae is measured after an interval of 24 hours. The jars containing the samples are characterized in texturometry using a texturometer such as the machine sold by the company Rheo TA-XT2, according to the following protocol: a stainless-steel ball probe 5 mm in diameter is brought into contact with the sample at a speed of 1 mm/s. The measuring system detects the interface with the sample, with a detection threshold equal to 0.005 newtons. The probe penetrates 0.3 mm into the sample, at a speed of 0.1 mm/s. The measuring machine records the change in force measured in compression over time, during the penetration phase. The hardness of the sample corresponds to the average of the maximum force values detected during penetration, over at least three measurements.

### AQUEOUS PHASE

The compositions according to the invention intended for cosmetic use may comprise at least one aqueous phase. They are especially formulated as aqueous lotions or as water-in-oil or oil-in-water emulsions or as multiple emulsions (oil-in-water-in-oil or water-in-oil-in-water triple emulsion (such emulsions are known and described, for example, by C. Fox in "Cosmetics and Toiletries" - November 1986 - Vol. 101 - pages 101-112)).

The aqueous phase of the said compositions contains water and generally other water-soluble or water-miscible solvents. The water-soluble or water-miscible solvents comprise monoalcohols with a short chain, for example of C₁-C₄, such as ethanol or isopropanol; diols or polyols, for instance ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol. Propylene glycol and glycerol, propane-1,3-diol, will be used more particularly.

### EMULSIFIERS

### a) Oil-in-water emulsifiers

As emulsifiers that may be used in the oil-in-water emulsions or oil-in-water-in-oil triple emulsions, examples that may be mentioned include nonionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters such as sucrose stearate; and mixtures thereof, such as the mixture of glyceryl stearate and PEG-40 stearate.

Mention may also be made of fatty alcohol/alkylpolyglycoside emulsifying mixtures as described in patent applications WO 92/06778, WO 95/13863 and WO 98/47610, for instance the commercial products sold by the company SEPPIC under the name Montanov®.

### b) Water-in-oil emulsifiers

Among the emulsifiers that may be used in the water-in-oil emulsions or water-in-oil-in-water-in-oil triple emulsions, examples that may be mentioned include alkyl dimethicone copolyols corresponding to formula (I) below 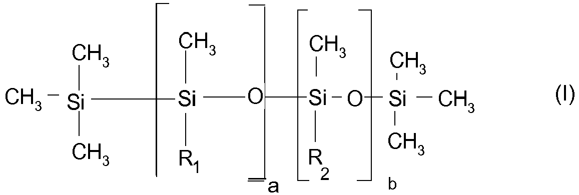 in which:
R₁ denotes a linear or branched C₁₂-C₂₀ and preferably C₁₂-C₁₈ alkyl group;
R₂ denotes the group: --CₙH₂ₙ--(-OC₂H₄-)ₓ--(-OC₃H₆-)_{y}--O-R₃,
R₃ denotes a hydrogen atom or a linear or branched alkyl radical comprising from 1 to 12 carbon atoms;
a is an integer ranging from 1 to 500;
b is an integer ranging from 1 to 500;
n is an integer ranging from 2 to 12 and preferably from 2 to 5;
x is an integer ranging from 1 to about 50 and preferably from 1 to 30;
y is an integer ranging from 0 to about 49 and preferably from 0 to 29, with the proviso that when y is other than zero, the ratio x/y is greater than 1 and preferably ranges from 2 to 11.

Among the alkyl dimethicone copolyol emulsifiers of formula (I) that are preferred, mention will be made more particularly of Cetyl PEG/PPG-10/1 Dimethicone and more particularly the mixture Cetyl PEG/PPG-10/1 Dimethicone and Dimethicone (INCI name), for instance the product sold under the trade name Abil EM90 by the company Goldschmidt, or alternatively the mixture (Polyglyceryl-4 Stearate and Cetyl PEG/PPG-10 (and) Dimethicone (and) Hexyl Laurate), for instance the product sold under the trade name Abil WE09 by the same company.

Among the water-in-oil emulsifiers, mention may also be made of the dimethicone copolyols corresponding to formula (II) below 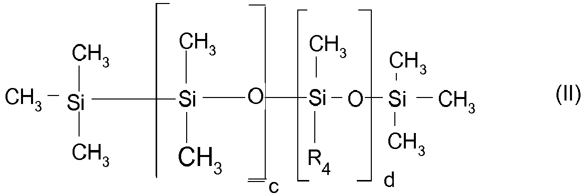 in which:
R₄ denotes the group: --CₘH₂ₘ--(-OC₂H₄-)ₛ--(-OC3H₆-)ₜ--O-R₅,
R₅ denotes a hydrogen atom or a linear or branched alkyl radical comprising from 1 to 12 carbon atoms,
c is an integer ranging from 1 to about 500;
d is an integer ranging from 1 to about 500;
m is an integer ranging from 2 to 12 and preferably from 2 to 5;
s is an integer ranging from 1 to about 50 and preferably from 1 to 30;
t is an integer ranging from 0 to about 50 and preferably from 0 to 30; with the proviso that the sum s+t is greater than or equal to 1.

Among these preferential dimethicone copolyol emulsifiers of formula (II), use will particularly be made of PEG-18/PPG-18 Dimethicone and more particularly the mixture Cyclopentasiloxane (and) PEG-18/PPG-18 Dimethicone (INCI name), such as the product sold by the company Dow Corning under the trade name Silicone DC5225 C or KF-6040 from the company Shin-Etsu.

According to one particularly preferred form, use will be made of a mixture of at least one emulsifier of formula (I) and of at least one emulsifier of formula (II).

Use will be made more particularly of a mixture of PEG-18/PPG-18 Dimethicone and Cetyl PEG/PPG-10/1 Dimethicone and even more particularly a mixture of (Cyclopentasiloxane (and) PEG-18/PPG-18 Dimethicone) and of Cetyl PEG/PPG-10/1 Dimethicone and Dimethicone or of (Polyglyceryl-4-stearate and Cetyl PEG/PPG-10 (and) Dimethicone (and) Hexyl Laurate).

Among the water-in-oil emulsifiers, mention may also be made of nonionic emulsifiers derived from fatty acids and polyol, alkylpolyglycosides (APG) and sugar esters, and mixtures thereof.

As nonionic emulsifiers derived from fatty acids and polyol, use may be made especially of fatty acid esters of polyol, the fatty acid especially containing a C8-C24 alkyl chain, and the polyols being, for example, glycerol and sorbitan.

Fatty acid esters of polyol that may especially be mentioned include isostearic acid esters of polyols, stearic acid esters of polyols, and mixtures thereof, in particular isostearic acid esters of glycerol and/or sorbitan.

Stearic acid esters of polyols that may especially be mentioned include the polyethylene glycol esters, for instance PEG-30 Dipolyhydroxystearate, such as the product sold under the name Arlacel P135 by the company ICI.

Glycerol and/or sorbitan esters that may be mentioned, for example, include polyglyceryl isostearate, such as the product sold under the name Isolan Gl 34 by the company Goldschmidt; sorbitan isostearate, such as the product sold under the name Arlacel 987 by the company ICI; sorbitan glyceryl isostearate, such as the product sold under the name Arlacel 986 by the company ICI, the mixture of sorbitan isostearate and polyglyceryl isostearate (3 mmol) sold under the name Arlacel 1690 by the company Uniqema, and mixtures thereof.

The emulsifier may also be chosen from alkylpolyglycosides with an HLB of less than 7, for example those represented by the general formula (1) below:

R-O-(G)x (1)

in which R represents a branched and/or unsaturated alkyl radical comprising from 14 to 24 carbon atoms, G represents a reduced sugar comprising 5 or 6 carbon atoms, and x is a value ranging from 1 to 10 and preferably from 1 to 4, and G especially denotes glucose, fructose or galactose.

The unsaturated alkyl radical may comprise one or more ethylenic unsaturations, and in particular one or two ethylenic unsaturations.

As alkylpolyglycosides of this type, mention may be made of alkylpolyglucosides (G = glucose in formula (I)), and especially the compounds of formula (I) in which R more particularly represents an oleyl radical (unsaturated C₁₈ radical) or isostearyl (saturated C₁₈ radical), G denotes glucose, x is a value ranging from 1 to 2, especially isostearyl glucoside or oleyl glucoside, and mixtures thereof. This alkylpolyglucoside may be used as a mixture with a coemulsifier, more especially with a fatty alcohol and especially a fatty alcohol containing the same fatty chain as that of the alkylpolyglucoside, i.e. comprising from 14 to 24 carbon atoms and containing a branched and/or unsaturated chain, for example isostearyl alcohol when the alkylpolyglucoside is isostearyl glucoside, and oleyl alcohol when the alkylpolyglucoside is oleyl glucoside, optionally in the form of a self-emulsifying composition, as described, for example, in document WO-A-92/06778. Use may be made, for example, of the mixture of isostearyl glucoside and isostearyl alcohol, sold under the name Montanov WO 18 by the company SEPPIC, and also the mixture octyldodecanol and octyldodecyl xyloside sold under the name Fludanov 20X by the company SEPPIC.

Mention may also be made of succinic-terminated polyolefins, for instance esterified succinic-terminated polyisobutylenes and salts thereof, especially the diethanolamine salts, such as the commercial products sold under the names Lubrizol 2724, Lubrizol 2722 and Lubrizol 5603 by the company Lubrizol or the commercial product Chemcinnate 2000.

The total amount of emulsifiers in the composition will preferably be, in the composition according to the invention, in active material contents ranging from 1% to 8% by weight and more particularly from 2% to 6% by weight relative to the total weight of the composition.

### FATTY PHASE

The compositions according to the invention may contain at least one water-immiscible organic liquid phase, known as a fatty phase. This phase generally comprises one or more hydrophobic compounds that make the said phase water-immiscible. The said phase is liquid (in the absence of structuring agent) at room temperature (20-25°C). Preferentially, the water-immiscible organic-liquid organic phase in accordance with the invention generally comprises at least one volatile oil and/or non-volatile oil and optionally at least one structuring agent.

The term "oil" means a fatty substance that is liquid at room temperature (25°C) and atmospheric pressure (760 mmHg, i.e. 105 Pa). The oil may be volatile or non-volatile.

For the purposes of the invention, the term "volatile oil" means an oil that is capable of evaporating on contact with the skin or the keratin fibre in less than one hour, at room temperature and atmospheric pressure. The volatile oils of the invention are volatile cosmetic oils, which are liquid at room temperature, having a non-zero vapour pressure, at room temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil that remains on the skin or the keratin fibre at room temperature and atmospheric pressure for at least several hours, and that especially has a vapour pressure of less than 10⁻³ mmHg (0.13 Pa).

The oil may be chosen from any physiologically acceptable oil and in particular cosmetically acceptable oil, especially mineral, animal, plant or synthetic oils; in particular volatile or nonvolatile hydrocarbon-based oils and/or silicone oils and/or fluoro oils, and mixtures thereof.

More precisely, the term "hydrocarbon-based oil" means an oil mainly comprising carbon and hydrogen atoms and optionally one or more functions chosen from hydroxyl, ester, ether and carboxylic functions. Generally, the oil has a viscosity of from 0.5 to 100 000 mPa.s, preferably from 50 to 50 000 mPa.s and more preferably from 100 to 300 000 mPa.s.

As examples of volatile oils that may be used in the invention, mention may be made of:
- volatile hydrocarbon-based oils chosen from hydrocarbon-based oils containing from 8 to 16 carbon atoms, and especially C₈-C₁₆ isoalkanes of petroleum origin (also known as isoparaffins), for instance isododecane (also known as 2,2,4,4,6-pentamethylheptane), isodecane and isohexadecane, for example the oils sold under the trade names Isopar or Permethyl, branched C₈-C₁₆ esters and isohexyl neopentanoate, and mixtures thereof. Other volatile hydrocarbon-based oils, for instance petroleum distillates, especially those sold under the name Shell Solt by the company Shell, may also be used; volatile linear alkanes, such as those described in patent application DE10 2008 012 457 from the company Cognis.
- volatile silicones, for instance volatile linear or cyclic silicone oils, especially those with a viscosity ≤ 8 centistokes (8×10⁻⁶ m²/s) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane;
- and mixtures thereof.

Mention may also be made of linear volatile alkyltrisiloxane oils of general formula (I): 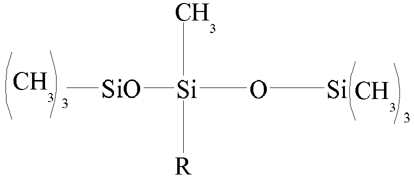 in which R represents an alkyl group containing from 2 to 4 carbon atoms, of which one or more hydrogen atoms may be substituted with a fluorine or chlorine atom.

Among the oils of general formula (I) that may be mentioned are:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

As examples of nonvolatile oils that may be used in the invention, mention may be made of:
- hydrocarbon-based oils of animal origin, such as perhydrosqualene;
- hydrocarbon-based plant oils such as liquid triglycerides of fatty acids of 4 to 24 carbon atoms, for instance heptanoic or octanoic acid triglycerides, or wheatgerm oil, olive oil, sweet almond oil, palm oil, rapeseed oil, cottonseed oil, alfalfa oil, poppy oil, pumpkin oil, marrow oil, blackcurrant oil, evening primrose oil, millet oil, barley oil, quinoa oil, rye oil, safflower oil, candlenut oil, passion flower oil, musk rose oil, sunflower oil, corn oil, soybean oil, grapeseed oil, sesame seed oil, hazelnut oil, apricot oil, macadamia oil, castor oil, avocado oil, caprylic/capric acid triglycerides, for instance those sold by the company Stéarineries Dubois or those sold under the names Miglyol 810, 812 and 818 by the company Sasol, jojoba oil and shea butter oil,
- linear or branched hydrocarbons, of mineral or synthetic origin, such as liquid paraffins and derivatives thereof, petroleum jelly, polydecenes, polybutenes, hydrogenated polyisobutene such as Parleam, and squalane,
- synthetic ethers containing from 10 to 40 carbon atoms;
- synthetic esters, especially of fatty acids, for instance the oils of formula R₁COOR₂ in which R₁ represents a linear or branched higher fatty acid residue containing from 1 to 40 carbon atoms and R₂ represents a hydrocarbon-based chain, which is especially branched, containing from 1 to 40 carbon atoms, with R₁ + R₂ ≥ 10, for instance purcellin oil (cetostearyl octanoate), isononyl isononanoate, isopropyl myristate, isopropyl palmitate, C₁₂-C₁₅ alkyl benzoates, hexyl laurate, diisopropyl adipate, isononyl isononanoate, 2-ethylhexyl palmitate, 2-octyldodecyl stearate, 2-octyldodecyl erucate, isostearyl isostearate or tridecyl trimellitate; alcohol or polyalcohol octanoates, decanoates or ricinoleates, for instance propylene glycol dioctanoate; hydroxylated esters, for instance isostearyl lactate, octyl hydroxystearate, octyldodecyl hydroxystearate, diisostearyl malate, triisocetyl citrate, and fatty alcohol heptanoates, octanoates or decanoates; polyol esters, for instance propylene glycol dioctanoate, neopentyl glycol diheptanoate or diethylene glycol diisononanoate; and pentaerythritol esters, for instance pentaerythrityl tetraisostearate,
- fatty alcohols that are liquid at room temperature, containing a branched and/or unsaturated carbon-based chain containing from 12 to 26 carbon atoms, for instance octyldodecanol, isostearyl alcohol, 2-butyloctanol, 2-hexyldecanol, 2-undecylpentadecanol or oleyl alcohol,
- higher fatty acids such as oleic acid, linoleic acid or linolenic acid;
- carbonates;
- acetates;
- citrates;
- fluoro oils that are optionally partially hydrocarbon-based and/or silicone-based, for instance fluorosilicone oils, fluoro polyethers and fluorosilicones as described in document EP-A-847 752;
- silicone oils, for instance linear or cyclic non-volatile polydimethylsiloxanes (PDMS); polydimethylsiloxanes comprising alkyl, alkoxy or phenyl groups, which are pendant or at the end of a silicone chain, these groups containing from 2 to 24 carbon atoms; phenyl silicones, for instance phenyl trimethicones, phenyl dimethicones, phenyl trimethylsiloxy diphenyl siloxanes, diphenyl dimethicones, diphenyl methyldiphenyl trisiloxanes and 2-phenylethyl trimethylsiloxy silicates, and
- mixtures thereof.

### STRUCTURING AGENT

The compositions according to the invention comprising a fatty phase may also contain at least one agent for structuring the said fatty phase, which may preferably be chosen from waxes, pasty compounds, and mineral or organic lipophilic gelling agents, and mixtures thereof.

It is understood that the amount of these compounds may be adjusted by a person skilled in the art so as not to harm the desired effect in the context of the present invention.

### Wax(es)

The wax is in general a lipophilic compound that is solid at room temperature (25°C), with a solid/liquid reversible change of state, having a melting point of greater than or equal to 30°C, which may be up to 200°C and in particular up to 120°C.

In particular, the waxes that are suitable for the invention may have a melting point of greater than or equal to 45°C and in particular greater than or equal to 55°C.

For the purposes of the invention, the melting point corresponds to the temperature of the most endothermic peak observed on thermal analysis (DSC) as described in standard ISO 11357-3; 1999. The melting point of the wax may be measured using a differential scanning calorimeter (DSC), for example the calorimeter sold under the name MDSC 2920 by the company TA Instruments.

### The measuring protocol is as follows:

A sample of 5 mg of wax placed in a crucible is subjected to a first temperature rise ranging from -20°C to 100°C, at a heating rate of 10°C/minute, it is then cooled from 100°C to -20°C at a cooling rate of 10°C/minute and is finally subjected to a second temperature increase ranging from -20°C to 100°C at a heating rate of 5°C/minute. During the second temperature increase, the variation of the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

The waxes that may be used in the compositions according to the invention are chosen from waxes that are solid at room temperature of animal, plant, mineral or synthetic origin, and mixtures thereof.

As illustrations of waxes that are suitable for the invention, mention may be made especially of hydrocarbon-based waxes, for instance beeswax, lanolin wax, Chinese insect waxes, rice bran wax, carnauba wax, candelilla wax, ouricury wax, esparto grass wax, berry wax, shellac wax, Japan wax and sumach wax; montan wax, orange wax and lemon wax, refined sunflower wax sold under the name Sunflower Wax by Koster Keunen, microcrystalline waxes, paraffins and ozokerite; polyethylene waxes, the waxes obtained by Fischer-Tropsch synthesis and waxy copolymers, and also esters thereof.

Mention may also be made of waxes obtained by catalytic hydrogenation of animal or plant oils containing linear or branched C₈-C₃₂ fatty chains. Among these waxes that may especially be mentioned are isomerized jojoba oil such as the trans-isomerized partially hydrogenated jojoba oil manufactured or sold by the company Desert Whale under the commercial reference Iso-Jojoba-50®, hydrogenated sunflower oil, hydrogenated castor oil, hydrogenated coconut oil, hydrogenated lanolin oil and bis(1,1,1-trimethylolpropane) tetrastearate sold under the name Hest 2T-4S® by the company Heterene.

Mention may also be made of silicone waxes (C₃₀₋₄₅ alkyl dimethicone) and fluoro waxes.

The waxes obtained by hydrogenation of castor oil esterified with cetyl alcohol, sold under the names Phytowax ricin 16L64® and 22L73® by the company Sophim, may also be used. Such waxes are described in patent application FR-A-2 792 190.

A wax that may be used is a C₂₀-C₄₀ alkyl (hydroxystearyloxy)stearate (the alkyl group containing from 20 to 40 carbon atoms), alone or as a mixture.

Such a wax is especially sold under the names Kester Wax K 82 P®, Hydroxypolyester K 82 P® and Kester Wax K 80 P® by the company Koster Keunen.

As microwaxes that may be used in the compositions according to the invention, mention may be made especially of carnauba microwaxes, such as the product sold under the name MicroCare 350® by the company Micro Powders, synthetic microwaxes, such as the product sold under the name MicroEase 114S® by the company Micro Powders, microwaxes consisting of a mixture of carnauba wax and polyethylene wax, such as the products sold under the names Micro Care 300® and 310® by the company Micro Powders, microwaxes consisting of a mixture of carnauba wax and of synthetic wax, such as the product sold under the name Micro Care 325® by the company Micro Powders, polyethylene microwaxes, such as the products sold under the names Micropoly 200®, 220®, 220L® and 250S® by the company Micro Powders, the commercial products Performalene 400, Polyethylene and Performalene 500-L Polyethylene from New Phase Technologies, Performalene 655, Polyethylene or paraffin waxes, for instance the wax having the INCI name Microcrystalline Wax and Synthetic Wax and sold under the trade name Microlease by the company Sochibo; polytetrafluoroethylene microwaxes such as those sold under the names Microslip 519® and 519 L® by the company Micro Powders.

The composition according to the invention may preferably comprise a content of wax(es) ranging from 3% to 20% by weight relative to the total weight of the composition, in particular from 5% to 15% and more particularly from 6% to 15% thereof.

According to one particular form of the invention, in the context of anhydrous solid compositions in stick form, use will be made of polyethylene microwaxes in the form of crystallites with an aspect ratio at least equal to 2, and with a melting point ranging from 70 to 110°C and preferably from 70 to 100°C, so as to reduce or even eliminate the presence of strata in the solid composition.

These crystallites in needle form and especially the dimensions thereof may be characterized visually according to the following method.

The wax is deposited on a microscope slide, which is placed on a hotplate. The slide and the wax are heated to a temperature generally at least 5°C higher than the melting point of the wax or of the mixture of waxes under consideration. At the end of melting, the liquid thus obtained and the microscope slide are allowed to cool to solidify. Observation of the crystallites is performed using a Leica DMLB100 optical microscope, with an objective lens selected as a function of the size of the objects to be viewed, and under polarized light. The dimensions of the crystallites are measured using image analysis software such as that sold by the company Microvision.

The crystallite polyethylene waxes in accordance with the invention preferably have an average length ranging from 5 to 10 µm. The term "average length" denotes the dimension given by statistical granulometric distribution of half the population, which is written as D50.

Use will be made more particularly of a mixture of the waxes Performalene 400 Polyethylene and Performalene 500-L Polyethylene from New Phase Technologies.

### Pasty compounds

For the purposes of the present invention, the term "pasty compound" is intended to denote a lipophilic fatty compound that undergoes a reversible solid/liquid change of state, which has in solid form an anisotropic crystal organization, and that comprises, at a temperature of 23°C, a liquid fraction and a solid fraction.

The pasty compound is preferably chosen from synthetic compounds and compounds of plant origin. A pasty compound may be obtained by the synthesis from starting materials of plant origin.

The pasty compound may be advantageously chosen from:
- lanolin and derivatives thereof,
- polymeric or non-polymeric silicone compounds,
- polymeric or non-polymeric fluoro compounds,
- vinyl polymers, especially:
- olefin homopolymers,
- olefin copolymers,
- hydrogenated diene homopolymers and copolymers,
- linear or branched oligomers, homopolymers or copolymers of alkyl (meth)acrylates preferably containing a C₈-C₃₀ alkyl group,
- oligomers, homopolymers and copolymers of vinyl esters containing C₈-C₃₀ alkyl groups,
- oligomers, homopolymers and copolymers of vinyl ethers containing C₈-C₃₀ alkyl groups,
- liposoluble polyethers resulting from the polyetherification between one or more C₂-C₁₀₀ and preferably C₂-C₅₀ diols,
- esters,
- mixtures thereof.

Among the esters, the following are especially preferred:
- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, especially such as those sold under the brand name Softisan 649 by the company Sasol,
- the arachidyl propionate sold under the brand name Waxenol 801 by Alzo,
- phytosterol esters,
- fatty acid triglycerides and derivatives thereof,
- pentaerythritol esters,
- non-crosslinked polyesters resulting from polycondensation between a linear or branched C₄-C₅₀ dicarboxylic acid or polycarboxylic acid and a C₂-C₅₀ diol or polyol,
- aliphatic esters of an ester, resulting from the esterification of an aliphatic hydroxycarboxylic acid ester with an aliphatic carboxylic acid,
- polyesters resulting from the esterification, with a polycarboxylic acid, of an aliphatic hydroxycarboxylic acid ester, the said ester comprising at least two hydroxyl groups, such as the products Risocast DA-H® and Risocast DA-L®,
- esters of a diol dimer and of a diacid dimer, where appropriate esterified on their free alcohol or acid function(s) with acid or alcohol radicals, such as Plandool-G,
- mixtures thereof.

Among the pasty compounds of plant origin that will preferably be chosen is a mixture of oxyethylenated (5 OE) oxypropylenated (5 OP) soybean sterols and pentaerythritol, sold under the reference Lanolide by the company Vevy.

### Lipophilic gelling agents

### Mineral gelling agents

Mineral lipophilic gelling agents that may be mentioned include optionally modified clays, for instance hectorites modified with a C₁₀-C₂₂ ammonium chloride, for instance hectorite modified with distearyldimethylammonium chloride, for instance the product sold under the name Bentone 38V® by the company Elementis.

Mention may also be made of fumed silica optionally subjected to a hydrophobic surface treatment, the particle size of which is less than 1 µm. Specifically, it is possible to chemically modify the surface of the silica, by chemical reaction generating a reduced number of silanol groups present at the surface of the silica. It is especially possible to substitute silanol groups with hydrophobic groups: a hydrophobic silica is then obtained. The hydrophobic groups may be trimethylsiloxyl groups, which are obtained especially by treating fumed silica in the presence of hexamethyldisilazane. Silicas thus treated are known as "silica silylate" according to the CTFA (8th Edition, 2000). They are sold, for example, under the references Aerosil R812® by the company Degussa, Cab-O-Sil TS-530® by the company Cabot, dimethylsilyloxyl or polydimethylsiloxane groups, which are obtained especially by treating fumed silica in the presence of polydimethylsiloxane or dimethyldichlorosilane. Silicas thus treated are known as "silica dimethyl silylate" according to the CTFA (8th Edition, 2000). They are sold, for example, under the references Aerosil R972® and Aerosil R974® by the company Degussa, and Cab-O-Sil TS-610® and Cab-O-Sil TS-720® by the company Cabot.

The hydrophobic fumed silica preferably has a particle size that may be nanometric to micrometric, for example ranging from about 5 to 200 nm.

### Organic gelling agents

The polymeric organic lipophilic gelling agents are, for example, partially or totally crosslinked elastomeric organopolysiloxanes of three-dimensional structure, for instance those sold under the names KSG6®, KSG16® and KSG18® from Shin-Etsu, Trefil E-505C® or Trefil E-506C® from Dow Corning, Gransil SR-CYC®, SR DMF10®, SR-DC556®, SR 5CYC gel®, SR DMF 10 gel® and SR DC 556 gel® from Grant Industries and SF 1204® and JK 113® from General Electric; ethylcellulose, for instance the product sold under the name Ethocel® by Dow Chemical; galactomannans comprising from one to six and in particular from two to four hydroxyl groups per saccharide, substituted with a saturated or unsaturated alkyl chain, for instance guar gum alkylated with C1 to C6, and in particular C1 to C3, alkyl chains, and mixtures thereof. Block copolymers of "diblock", "triblock" or "radial" type, of the polystyrene/polyisoprene or polystyrene/polybutadiene type, such as the products sold under the name Luvitol HSB® by the company BASF, of the polystyrene/copoly(ethylene-propylene) type, such as the products sold under the name Kraton® by the company Shell Chemical Co., or of the polystyrene/copoly(ethylene-butylene) type, and mixtures of triblock and radial (star) copolymers in isododecane, such as those sold by the company Penreco under the name Versagel®, for instance the mixture of butylene/ethylene/styrene triblock copolymer and of ethylene/propylene/styrene star copolymer in isododecane (Versagel M 5960).

Lipophilic gelling agents that may also be mentioned include polymers with a weight-average molecular mass of less than 100 000, comprising a) a polymer backbone with hydrocarbon-based repeating units containing at least one heteroatom, and optionally b) at least one optionally functionalized pendent fatty chain and/or terminal fatty chain, containing from 6 to 120 carbon atoms and being linked to these hydrocarbon-based units, as described in patent applications WO-A-02/056 847 and WO-A-02/47619, in particular polyamide resins (especially comprising alkyl groups containing from 12 to 22 carbon atoms) such as those described in US-A-5 783 657.

Among the lipophilic gelling agents that may be used in the compositions according to the invention, mention may also be made of fatty acid esters of dextrin, such as dextrin palmitates, especially the products sold under the name Rheopearl TL® or Rheopearl KL® by the company Chiba Flour.

Silicone polyamides of the polyorganosiloxane type may also be used, such as those described in documents US-A-5 874 069, US-A-5 919 441, US-A-6 051 216 and US-A-5 981 680.

These silicone polymers may belong to the following two families:
- polyorganosiloxanes comprising at least two groups capable of establishing hydrogen interactions, these two groups being in the chain of the polymer, and/or
- polyorganosiloxanes comprising at least two groups capable of establishing hydrogen interactions, these two groups being located on grafts or branches.

### MOISTURE ABSORBERS

It is also possible to add moisture absorbers, for instance perlites and preferably expanded perlites.

The perlites that may be used according to the invention are generally aluminosilicates of volcanic origin and have as the composition:
70.0-75.0% by weight of silica SiO₂
12.0-15.0% by weight of oxide of aluminium oxide Al₂O₃
3.0-5.0% of sodium oxide Na₂O
3.0-5.0% of potassium oxide K₂O
0.5-2% of iron oxide Fe₂O₃ →
0.2-0.7% of magnesium oxide MgO
0.5-1.5% of calcium oxide CaO
0.05-0.15% of titanium oxide TiO₂

The perlite is ground, dried and then calibrated in a first step. The product obtained, known as perlite ore, is grey-coloured and has a size of about 100 µm.

The perlite ore is then expanded (1000°C/2 seconds) to give more or less white particles. When the temperature reaches 850-900°C, the water trapped in the structure of the material evaporates and brings about the expansion of the material relative to its original volume. The expanded perlite particles in accordance with the invention may be obtained via the expansion process described in patent US 5 002 698.

Preferably, the perlite particles used are ground: in this case, they are known as Expanded Milled Perlite (EMP). They preferably have a particle size defined by a median diameter D₅₀ ranging from 0.5 to 50 µm and preferably from 0.5 to 40 µm.

Preferably, the perlite particles used have an untamped apparent density at 25°C ranging from 10 to 400 kg/m³ (standard DIN 53468) and preferably from 10 to 300 kg/m³.

Preferably, the expanded perlite particles according to the invention have a water-absorbing capacity, measured at the wet point, ranging from 200% to 1500% and preferably from 250% to 800%.

The wet point corresponds to the amount of water that needs to be added to 1 g of particle in order to obtain a homogeneous paste. This method is derived directly from that of the oil uptake applied to solvents. The measurements are taken in the same manner by means of the wet point and the flow point, which have, respectively, the following definition:
wet point: mass expressed in grams per 100 g of product corresponding to the production of a homogeneous paste during the addition of a solvent to a powder;
flow point: mass expressed in grams per 100 g of product at and above which the amount of solvent is greater than the capacity of the powder to retain it. This is reflected by the production of a more or less homogeneous mixture that flows on a glass plate.

The wet point and the flow point are measured according to the following protocol:

### Protocol for measuring the water absorption

### 1) Materials used

Glass plate (25 x 25 mm)
Spatula (wooden and partly metallic handle, 15 x 2.7 mm)
Silk-bristled brush
Balance

### 2) Procedure

The glass plate is placed on the balance and 1 g of perlite particles is weighed out. The beaker containing the solvent and the sampling pipette is placed on the balance. The solvent is gradually added to the powder, the whole being regularly blended (every 3 to 4 drops) with the spatula.
The mass of solvent needed to obtain the wet point is noted. Further solvent is added and the mass required to reach the flow point is noted. The average of three tests is determined.

The expanded perlite particles sold under the trade names Optimat 1430 OR or Optimat 2550 by the company World Minerals will be used in particular.

### DEODORANTS

The deodorant active agents may be bacteriostatic agents or bactericides that act on underarm odour microorganisms, such as 2,4,4'-trichloro-2'-hydroxydiphenyl ether (®Triclosan), 2,4-dichloro-2'-hydroxydiphenyl ether, 3',4',5'-trichlorosalicylanilide, 1-(3',4'-dichlorophenyl)-3-(4'-chlorophenyl)urea (®Triclocarban) or 3,7,11-trimethyldodeca-2,5,10-trienol (®Farnesol); quaternary ammonium salts such as cetyltrimethylammonium salts, cetylpyridinium salts, DPTA (1,3-diaminopropanetetraacetic acid), 1,2-decanediol (Symclariol from the company Symrise), glycerol derivatives, for instance caprylic/capric glycerides (Capmul MCM from Abitec), glyceryl caprylate or caprate (Dermosoft GMCY and Dermosoft GMC, respectively from Straetmans), Polyglyceryl-2 caprate (Dermosoft DGMC from Straetmans), and biguanide derivatives, for instance polyhexamethylene biguanide salts. - chlorhexidine and salts thereof; 4-phenyl-4,4-dimethyl-2-butanol (Symdeo MPP from Symrise).
Among the deodorant active agents in accordance with the invention, mention may also be made of - zinc salts, for instance zinc salicylate, zinc gluconate, zinc pidolate; zinc sulfate, zinc chloride, zinc lactate, zinc phenolsulfonate; salicylic acid and derivatives thereof such as 5-n-octanoylsalicylic acid.

The deodorant active agents may be odour absorbers such as zinc ricinoleate, sodium bicarbonate; metallic or non-metallic zeolites, cyclodextrins or alum.

It may also be a chelating agent such as Dissolvine GL-47-S from Akzo Nobel, EDTA; DPTA.
It may also be a polyol such as glycerol or propane-1,3-diol (Zemea Propane diol sold by Dupont Tate and Lyle Bioproducts).

Alternatively, it may be an enzyme inhibitor such as triethyl citrate.

## Claims

1. Cosmetic use of a flocculant polymer comprising as side chain non-quaternized pyridine groups directly or indirectly linked to the main chain as antiperspirant active agent, in which the flocculant polymer is chosen from homopolymers or copolymers comprising at least one monomer (C) to (F) defined below: 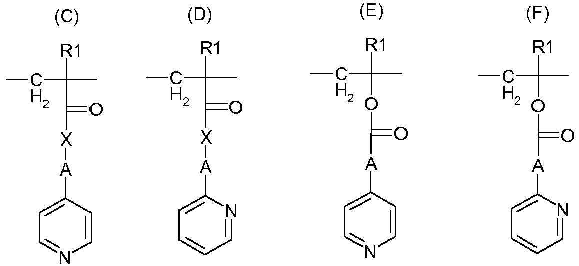 where:
X =O, or NH
R₁ represents a hydrogen atom or an alkyl group containing from 1 to 3 carbon atoms, preferably methyl;
A is a linear or branched C₁-C₆ and preferably C₂-C₃ alkyl group; a C₁-C₄ hydroxyalkyl group, preferably monohydroxyalkyl.

2. Use according to Claim 1, in which the flocculant polymer is contained in a composition comprising a cosmetically acceptable medium, in particular not containing any antiperspirant aluminium and/or zirconium salts.

3. Use according to Claim 2, in which the pyridine monomers (C) to (F) are chosen from N-(4-pyridyl)propylmethacrylamide and N-4-(pyridyl)ethyl methacrylate.

4. Use according to anyone of claims 1 to 3, in which the flocculant polymer also comprises units consisting of at least one cationic monomer other than a pyridine unit, which may be chosen from quaternary ammonium monomers and (meth)acrylamide monomers, or mixtures thereof.

5. Use according to claim 4, in which the additional cationic monomers are chosen from monomers having the following formulae: 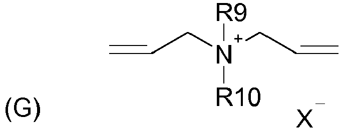 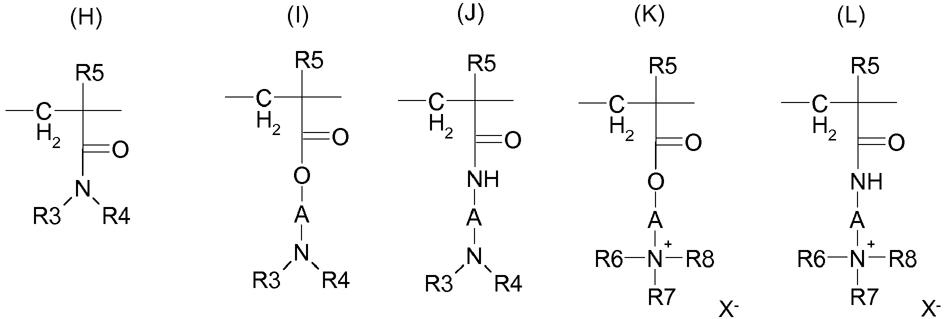 in which:
Rg and R₁₀ independently represent a linear or branched C₁-C₈ alkyl group, a benzyl group, a C₁-C₅ hydroxyalkyl group or a linear or branched amido(C₁-C₄)alkyl group;
R₃ and R₄ independently represent a hydrogen atom or a linear or branched alkyl group having from 1 to 6 carbon atoms, and preferably methyl or ethyl;
R₃ and R₄ may form with the nitrogen atom a 5- to 8-membered non-aromatic nitrogenous heterocycle, for instance pyrrolidine, piperazine or piperidine;
R₅ represents a hydrogen atom or an alkyl group containing from 1 to 3 carbon atoms, preferably methyl;
A is a linear or branched C₁-C₆ and preferably C₂-C₃ alkyl group; a C₁-C₄ hydroxyalkyl group, preferably monohydroxyalkyl;
R₆, R₇ and R₈, which may be identical or different, represent a hydrogen atom or a linear or branched C₁-C₆ alkyl group or a benzyl radical;
X denotes a mineral anion such as a halide (chloride, bromide or iodide) or an organic anion such as a (C₁-C₄) alkyl sulfate (methyl sulfate or ethyl sulfate).

6. Use according to Claim 4 and 5, in which the the additional cationic monomers are chosen from diallyldimethylammonium chloride (DADMAC), diallyldiethylammonium chloride (DADEAC), methacrylamide, acrylamide, methylaminoethyl methacrylate, benzyl dimethylaminoethylacrylate chloride (DMAEA-BCQ), acryloyloxyethyltrimethylammonium chloride (AETAC); methacryloyloxyethyltrimethylammonium chloride (METAC); methylacrylamidopropyltrimethylammonium chloride (MAPTAC); acrylamidopropyltrimethylammonium chloride (APTAC); acryloyloxyethyltrimethylammonium methosulfate (AETAMS); methacryloyloxyethyltrimethylammonium methosulfate (METAMS); acryloyloxyethyldiethylmethylammonium chloride; methacryloyloxyethyldiethylmethylammonium chloride or the equivalents thereof neutralized with a methyl sulfate, vinylimidazole, piperidine ethylmethacrylate and piperazine ethylmethacrylate.

7. Use according to claim 6, in which the additional cationic monomers are chosen from benzyl dimethylaminoethylacrylate chloride (DMAEA-BCQ), acryloyloxyethyltrimethylammonium chloride (AETAC), methacryloyloxyethyltrimethylammonium methosulfate (METAMS) and methylaminoethyl methacrylate.

8. Use according to anyone of claims 1 to 7, in which the flocculant polymer further comprises units consisting of at least one anionic monomer preferably chosen from those having the following formulae: 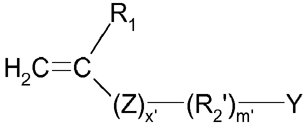 with
Y is a group chosen from -COOH, -SO₃H, -OSO₃H, -PO₃H₂ and -OPO₃H₂ wherein the groups SO₄H₂ and PO₄H₂ are linked to R'₂ via the oxygen atom, whereas the groups SO₃H and PO₃H are linked to R'₂, respectively, via the atoms S and P,
- R₁ represents a hydrogen atom or an alkyl group containing from 1 to 3 carbon atoms, preferably methyl,
- Z is a divalent group chosen from -COO-, -CONH-, -CONCH₃-, -OCO- or -O-, -SO₂- -CO-O-CO- or -CO-CH₂-CO-, preferably Z is chosen from COO and CONH,
- x' = 0 or 1, preferably 1,
- R'₂ is a saturated or unsaturated, optionally aromatic, linear, branched or cyclic carbon-based divalent radical of 1 to 30 carbon atoms, which may comprise 1 to 18 heteroatoms chosen from O, N, S, F, Si and P; the heteroatom(s) may be intercalated in the chain of the said radical R'₂ or alternatively the said radical R'₂ may be substituted with one or more groups comprising them such as hydroxyl or amino.

9. Use according to claim 8, in which the anionic monomer is chosen from maleic anhydride, acrylic acid, methacrylic acid, crotonic acid, itaconic acid, fumaric acid, maleic acid, 2-carboxyethyl acrylate (CH₂=CH-C(O)-O-(CH₂)₂-COOH); styrenesulfonic acid, 2-acrylamido-2-methylpropanesulfonic acid, vinylbenzoic acid, vinylphosphoric acid and sulfopropyl (meth)acrylate, and salts thereof.

10. Use according to anyone of claims 1 to 9, in which the flocculant polymer also comprises units consisting of at least one nonionic monomer chosen especially from those having the following formula, alone or as a mixture: 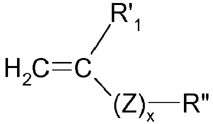 in which:
- R'₁ is hydrogen or -CH₃;
- Z is a divalent group chosen from -COO-, -CONH-, -CONCH₃-, -OCO-,
- SO₂, -CO-O-CO-, -CO-CH₂-CO- and -O-; preferably COO or CONH;
- x is 0 or 1;
- R" is a saturated or unsaturated, optionally aromatic, linear, branched or cyclic carbon-based radical of 1 to 30 carbon atoms, which may comprise 1 to 18 heteroatoms chosen from O, N, S, F, Si and P; the heteroatom(s) may be intercalated in the chain of the said radical or alternatively the said radical R" may be substituted with one or more groups comprising them such as hydroxyl, ester, amide, urethane or urea.

11. Use according to claim 10, in which the nonionic monomer is chosen from methyl methacrylate, methyl acrylate, ethyl methacrylate, ethyl acrylate, propyl acrylate, isopropyl acrylate, tetrahydrofurfuryl methacrylates, butyl methacrylate, 2-ethylhexyl acrylate, stearyl methacrylate, acrolein, tetrahydrofurfuryl acrylate, 2-hydroxyethyl methacrylate, 2-hydroxyethyl acrylate, ethoxyethyl methacrylate, ethoxyethyl acrylate, N-isopropylacrylamide, N-isopropylmethacrylamide, N,N-dimethylacrylamide, N,N-dimethylmethacrylamide, vinyl acetate, methyl vinyl ether, ethyl vinyl ether, vinylpyrrolidone, vinylcaprolactam, N-vinylacetamide, hydroxypropyl acrylate, N-vinyllactam, acrylamide, N-methylacrylamide, N,N-dimethylacrylamide, N-methyl-N-vinylacetamide, N-vinylformamide, N-methyl-N-vinylformamide, vinyl alcohol (copolymerized in the form of vinyl acetate and then hydrolysed).

12. Use according to any one of Claims 1 to 11, in which the flocculant polymer is chosen from:
- poly(4- or 2-vinylpyridine)-co-styrene copolymers;
- poly(4- or 2-vinylpyridine)-co-butyl methacrylate copolymers;
- poly(4- or 2-vinylpyridine) crosslinked with divinylbenzene;
- diblock polymers comprising a polyethylene glycol (PEG) or polyisobutylene block and a poly(4- or 2-vinylpyridine) block;
- poly(4-vinylpyridinium) p-toluenesulfonate;
- poly(4-vinylpyridine hydrochloride) 2% crosslinked with divinylbenzene.

13. Cosmetic process for treating perspiration, which consists in applying to the surface of the skin a composition comprising, in a cosmetically acceptable medium, at least one flocculant polymer as defined in any one of Claims 1 to 12, not containing any antiperspirant aluminium and/or zirconium salts.

## Patentansprüche

1. Kosmetische Verwendung eines Flockungspolymers, das als Seitenkette nicht quaternisierte Pyridingruppen, die direkt oder indirekt an die Hauptkette gebunden sind, umfasst, als Antiperspirant-Wirkstoff, wobei das Flockungspolymer aus Homopolymeren oder Copolymeren, die mindestens ein nachstehend definiertes Monomer (C) bis (F) umfassen, ausgewählt ist: 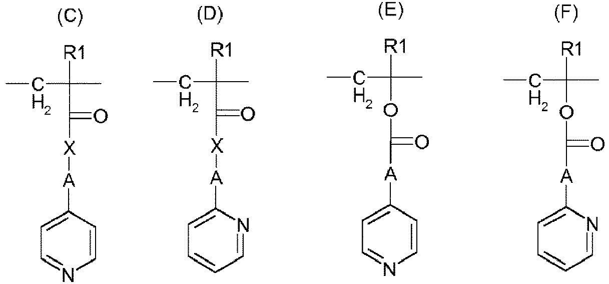 wobei:
X = 0 oder NH,
R₁ für ein Wasserstoffatom oder eine Alkylgruppe mit 1 bis 3 Kohlenstoffatomen, vorzugsweise Methyl, steht;
A für eine lineare oder verzweigte C₁-C₆- und vorzugsweise C₂-C₃-Alkylgruppe, oder eine C₁-C₄-Hydroxyalkylgruppe, vorzugsweise Monohydroxyalkyl, steht.

2. Verwendung nach Anspruch 1, wobei das Flockungspolymer in einer Zusammensetzung enthalten ist, die ein kosmetisch unbedenkliches Medium umfasst und insbesondere keine schweißhemmenden Aluminium- und/oder Zirconiumsalze enthält.

3. Verwendung nach Anspruch 2, wobei die Pyridin-Monomere (C) bis (F) aus N-(4-Pyridyl)propylmeth-acrylamid und N-4-(Pyridyl)ethylmethacrylat ausgewählt sind.

4. Verwendung nach einem der Ansprüche 1 bis 3, wobei das Flockungspolymer außerdem Einheiten umfasst, die aus mindestens einem kationischen Monomer bestehen, das von einer Pyridin-Einheit verschieden ist und aus quartären Ammonium-Monomeren und (Meth)acrylamid-Monomeren oder Mischungen davon ausgewählt sein kann.

5. Verwendung nach Anspruch 4, wobei die zusätzlichen kationischen Monomere aus Monomeren der folgenden Formel ausgewählt sind: 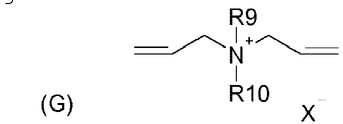 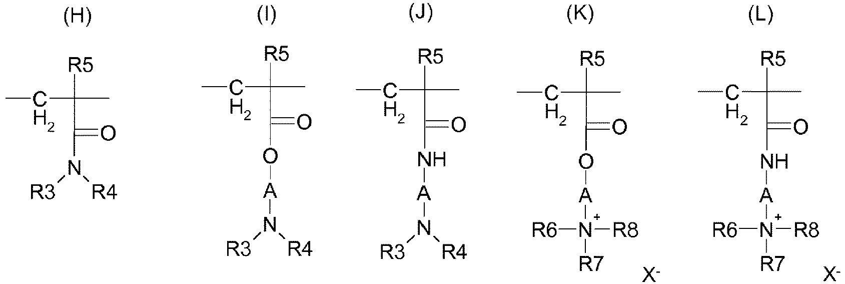 wobei:
R₉ und R₁₀ unabhängig für eine lineare oder verzweigte C₁-C₈-Alkylgruppe, eine Benzylgruppe, eine C₁-C₅-Hydroxyalkylgruppe oder eine lineare oder verzweigte Amido(C₁-C₄)alkylgruppe stehen,
R₃ und R₄ unabhängig für ein Wasserstoffatom oder eine lineare oder verzweigte Alkylgruppe mit 1 bis 6 Kohlenstoffatomen und vorzugsweise Methyl oder Ethyl stehen,
R₃ und R₄ mit dem Stickstoffatom einen 5- bis 8-gliedrigen nichtaromatischen stickstoffhaltigen Heterocyclus, beispielsweise Pyrrolidin, Piperazin oder Piperidin, bilden,
R₅ für ein Wasserstoffatom oder eine Alkylgruppe mit 1 bis 3 Kohlenstoffatomen, vorzugsweise Methyl, steht,
A für eine lineare oder verzweigte C₁-C₆- und vorzugsweise C₂-C₃-Alkylgruppe, oder eine C₁-C₄-Hydroxyalkylgruppe, vorzugsweise Monohydroxyalkyl, steht,
R₆, R₇ und R₈, die gleich oder verschieden sein können, für ein Wasserstoffatom oder eine lineare oder verzweigte C₁-C₆-Alkylgruppe oder einen Benzylrest stehen,
X für ein mineralisches Anion wie ein Halogenid (Chlorid, Bromid oder Iodid) oder ein organisches Anion wie ein (C₁-C₄)-Alkylsulfonat (Methylsulfat oder Ethylsulfat) steht.

6. Verwendung nach Anspruch 4 und 5, wobei die zusätzlichen kationischen Monomere aus Diallyldimethylammoniumchlorid (DADMAC), Diallyldiethylammoniumchlorid (DADEAC), Methacrylamid, Acrylamid, Methylaminoethylmethacrylat, Benzyldimethylaminoethylacrylatchlorid (DMAEA-BCQ), Acryloyloxyethyltrimethylammoniumchlorid (AETAC), Methacryloyloxyethyltrimethylammoniumchlorid (METAC), Methylacrylamidopropyltrimethylammoniumchlorid (MAPTAC), Acrylamidopropyltrimethylammoniumchlorid (APTAC), Acryloyloxyethyltrimethylammoniummethosulfat (AETAMS), Methacryloyloxyethyltrimethylammoniummethosulfat (METAMS), Acryloyloxyethyldiethylmethylammoniumchlorid, Methacryloyloxyethyldiethylmethylammoniumchlorid oder den mit einem Methylsulfat neutralisierten Äquivalenten davon, Vinylimidazol, Piperidinethylmethacrylat und Piperazinethylmethacrylat ausgewählt sind.

7. Verwendung nach Anspruch 6, wobei die zusätzlichen kationischen Monomere aus Benzyldimethylaminoethylacrylatchlorid (DMAEA-BCQ), Acryloyloxyethyltrimethylammoniumchlorid (AETAC), Methacryloyloxyethyltrimethylammoniummethosulfat (METAMS) und Methylaminoethylmethacrylat ausgewählt sind.

8. Verwendung nach einem der Ansprüche 1 bis 7, wobei das Flockungspolymer ferner Einheiten umfasst, die aus mindestens einem anionischen Monomer bestehen, das vorzugsweise aus denjenigen mit den folgenden Formeln ausgewählt ist: 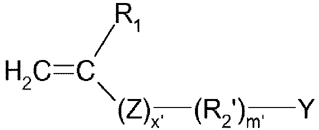 wobei
Y für eine Gruppe, die aus -COOH, -SO₃H, -OSO₃H,
- PO₃H₂ und -OPO₃H₂ ausgewählt ist, steht, wobei die Gruppen SO₄H₂ und PO₄H₂ über das Sauerstoffatom an R'₂ gebunden sind, wohingegen die Gruppen SO₃H und PO₃H über die Atome S bzw. P an R'₂ gebunden sind,
- R₁ für ein Wasserstoffatom oder eine Alkylgruppe mit 1 bis 3 Kohlenstoffatomen, vorzugsweise Methyl, steht,
- Z für eine zweiwertige Gruppe, die aus -COO-,
- CONH-, -CONCH₃-, -OCO- oder -O-, -SO₂-CO-O-CO-oder -CO-CH₂-CO- ausgewählt ist, steht, vorzugsweise Z aus COO und CONH ausgewählt ist,
- x' = 0 oder 1, vorzugsweise 1,
- R'₂ für einen gesättigten oder ungesättigten, gegebenenfalls aromatischen, linearen, verzweigten oder cyclischen zweiwertigen Rest auf Kohlenstoffbasis mit 1 bis 30 Kohlenstoffatomen steht, der 1 bis 18 Heteroatome, die aus 0, N, S, F, Si und P ausgewählt sind, enthalten kann, wobei das Heteroatom bzw. die Heteroatome in die Kette des Rests R'₂ eingeschoben sein kann bzw. können oder alternativ dazu der Rest R'₂ durch eine oder mehrere Gruppen, die sie umfassen, wie Hydroxyl oder Amino, substituiert sein kann.

9. Verwendung nach Anspruch 8, wobei das anionische Monomer aus Maleinsäureanhydrid, Acrylsäure, Methacrylsäure, Crotonsäure, Itaconsäure, Fumarsäure, Maleinsäure, 2-Carboxyethylacrylat (CH₂₌CH-C(O)-O-(CH₂)₂-COOH), Styrolsulfonsäure, 2-Acrylamido-2-methylpropansulfonsäure, Vinylbenzoesäure, Vinylphosphorsäure und Sulfopropyl(meth)-acrylat und Salzen davon ausgewählt ist.

10. Verwendung nach einem der Ansprüche 1 bis 9, wobei das Flockungspolymer außerdem Einheiten umfasst, die aus mindestens einem nichtionischen Monomer bestehen, das insbesondere aus denjenigen mit der folgenden Formel alleine oder als Mischung ausgewählt ist: 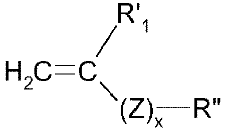 wobei
- R'₁ für Wasserstoff oder -CH₃ steht,
- Z für eine zweiwertige Gruppe, die aus -COO-,
- CONH-, -CONCH₃-, -OCO-, -SO₂, -CO-O-CO-, -CO-CH₂-CO- und -0- ausgewählt ist, vorzugsweise COO oder CONH, steht,
- x für 0 oder 1 steht,
- R'' für einen gesättigten oder ungesättigten, gegebenenfalls aromatischen, linearen, verzweigten oder cyclischen Rest auf Kohlenstoffbasis mit 1 bis 30 Kohlenstoffatomen steht, der 1 bis 18 Heteroatome, die aus 0, N, S, F, Si und P ausgewählt sind, enthalten kann, wobei das Heteroatom bzw. die Heteroatome in die Kette des Rests eingeschoben sein kann bzw. können oder alternativ dazu der Rest R'' durch eine oder mehrere Gruppen, die sie umfassen, wie Hydroxyl, Ester, Amid, Urethan oder Harnstoff, substituiert sein kann.

11. Verwendung nach Anspruch 10, wobei das nichtionische Monomer aus Methylmethacrylat, Methylacrylat, Ethylmethacrylat, Ethylacrylat, Propylacrylat, Isopropylacrylat, Tetrahydrofurfurylmethacrylat, Butylmethacrylat, 2-Ethylhexylacrylat, Stearylmethacrylat, Acrolein, Tetrahydrofurfurylacrylat, 2-Hydroxyethylmethacrylat, 2-Hydroxyethylacrylat, Ethoxyethylmethacrylat, Ethoxyethylacrylat, N-Isopropylacrylamid, N-Isopropylmethacrylamid, N,N-Dimethylacrylamid, N,N-Dimethylmethacrylamid, Vinylacetat, Methylvinylether, Ethylvinylether, Vinylpyrrolidon, Vinylcaprolactam, N-Vinylacetamid, Hydroxypropylacrylat, N-Vinyllactam, Acrylamid, N-Methylacrylamid, N,N-Dimethylacrylamid, N-Methyl-N-vinylacetamid, N-Vinylformamid, N-Methyl-N-vinylformamid, Vinylalkohol (copolymerisiert in Form von Vinylacetat und dann hydrolysiert) ausgewählt ist.

12. Verwendung nach einem der Ansprüche 1 bis 11, wobei das Flockungspolymer aus
- Poly(4- oder 2-vinylpyridin)-co-styrol-Copolymeren,
- Poly(4- oder 2-vinylpyridin)-co- butylmethacrylat-Copolymeren,
- mit Divinylbenzol vernetztem Poly(4- oder 2-vinylpyridin),
- Diblockpolymeren mit einem Polyethylenglykol-(PEG)- oder Polyisobutylen-Block und einem Poly(4- oder 2-vinylpyridin)-Block,
- Poly(4-vinylpyridinium)-p-toluolsulfonat,
- Poly(4-vinylpyridin-hydrochlorid) 2 % vernetzt mit Divinylbenzol
ausgewählt ist.

13. Kosmetisches Verfahren zur Behandlung von Perspiration, das darin besteht, dass man auf die Oberfläche der Haut eine Zusammensetzung aufbringt, die in einem kosmetisch unbedenklichen Medium mindestens ein Flockungspolymer gemäß einem der Ansprüche 1 bis 12 umfasst und keine schweißhemmenden Aluminium- und/oder Zirconiumsalze enthält.

## Revendications

1. Utilisation cosmétique d'un polymère floculant comprenant en tant que chaîne latérale des groupes pyridine non quaternisés directement ou indirectement liés à la chaîne principale en tant qu'agent actif antitranspirant, dans laquelle le polymère floculant est choisi parmi des homopolymères et des copolymères comprenant au moins un monomère (C) à (F) défini ci-dessous : 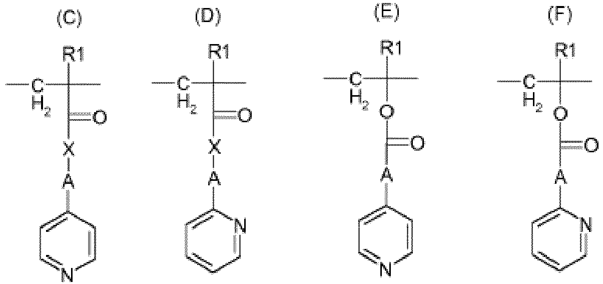 où :
X = 0, ou NH
R₁ représente un atome d'hydrogène ou un groupe alkyle contenant de 1 à 3 atome(s) de carbone, préférablement méthyle ;
A est un groupe C₁₋₆-alkyle et préférablement C₂₋₃-alkyle, linéaire ou ramifié ; un groupe C₁₋₄-hydroxyalkyle, préférablement monohydroxyalkyle.

2. Utilisation selon la revendication 1, dans laquelle le polymère floculant est contenu dans une composition comprenant un milieu acceptable sur le plan cosmétique, en particulier ne contenant aucun sels d'aluminium et/ou de zirconium antitranspirants.

3. Utilisation selon la revendication 2, dans laquelle les monomères de type pyridine (C) à (F) sont choisis parmi le N-(4-pyridinyl)propylméthacrylamide et le méthacrylate de N-4-(pyridinyl)éthyle.

4. Utilisation selon l'une quelconque des revendications 1 à 3, dans laquelle le polymère floculant comprend également des motifs constitués d'au moins un monomère cationique différent d'un motif de type pyridine, qui peut être choisi parmi des monomères de type ammonium quaternaire et des monomères de type (méth)acrylamide, ou des mélanges correspondants.

5. Utilisation selon la revendication 4, dans laquelle les monomères cationiques supplémentaires sont choisis parmi des monomères possédant les formules suivantes : 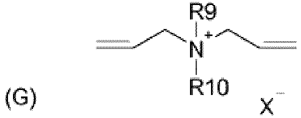 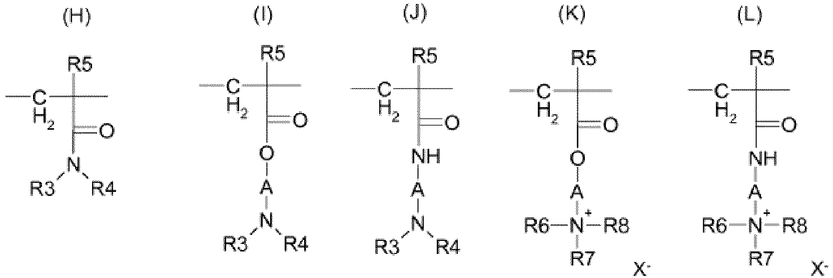 dans lesquelles :
R₉ et R₁₀ représentent indépendamment un groupe C₁₋₈-alkyle linéaire ou ramifié, un groupe benzyle, un groupe C₁₋₅-hydroxyalkyle ou un groupe amido-C₁₋₄-alkyle linéaire ou ramifié ;
R₃ et R₄ représentent indépendamment un atome d'hydrogène ou un groupe alkyle linéaire ou ramifié possédant de 1 à 6 atome(s) de carbone, et préférablement méthyle ou éthyle ;
R₃ et R₄ peuvent former avec l'atome d'azote un hétérocycle azoté non aromatique à 5 à 8 chaînons, par exemple pyrrolidine, pipérazine ou pipéridine ;
R₅ représente un atome d'hydrogène ou un groupe alkyle contenant de 1 à 3 atome(s) de carbone, préférablement méthyle ;
A est un groupe C₁₋₆-alkyle et préférablement C₂₋₃-alkyle, linéaire ou ramifié ; un groupe C₁₋₄-hydroxyalkyle, préférablement monohydroxyalkyle ;
R₆, R₇ et R₈, qui peuvent être identiques ou différents, représentent un atome d'hydrogène ou un groupe C₁₋₆-alkyle linéaire ou ramifié ou un radical benzyle ;
X désigne un anion minéral tel qu'un halogénure (chlorure, bromure ou iodure) ou un anion organique tel qu'un C₁₋₄-alkylsulfate (méthylsulfate ou éthylsulfate).

6. Utilisation selon les revendications 4 et 5, dans laquelle les monomères cationiques supplémentaires sont choisis parmi le chlorure de diallydiméthylammonium (DADMAC), le chlorure de diallydiéthylammonium (DADEAC), le méthacrylamide, l'acrylamide, le méthacrylate de méthylaminométhyle, le chlorure de diméthylaminoéthylacrylate de benzyle (DMAEA-BCQ), le chlorure d'acryloyloxyéthyltriméthylammonium (AETAC) ; le chlorure de méthacryloyloxyéthyltriméthylammonium (METAC) ; le chlorure de méthylacrylamidopropyltriméthylammonium (MAPTAC) ; le chlorure d'acrylamidopropyltriméthylammonium (APTAC) ; le méthosulfate d'acryloyloxyéthyltriméthylammonium (AETAMS) ; le méthosulfate de méthacryloyloxyéthyltriméthylammonium (METAMS) ; le chlorure d'acryloyloxyéthyldiéthylméthylammonium ; le chlorure de méthacryloyloxyéthyldiéthylméthylammonium ou les équivalents correspondants neutralisés avec un méthylsulfate, un vinyle imidazole, un pipéridine éthylméthacrylate et un pipérazine éthylméthacrylate.

7. Utilisation selon la revendication 6, dans laquelle les monomères cationiques supplémentaires sont choisis parmi le chlorure de diméthylaminoéthylacrylate de benzyle (DMAEA-BCQ), le chlorure d'acryloyloxyéthyltriméthylammonium (AETAC), le méthosulfate de méthacryloyloxyéthyltriméthylammonium (METAMS) et le méthacrylate de méthylaminoéthyle.

8. Utilisation selon l'une quelconque des revendications 1 à 7, dans laquelle le polymère floculant comprend en outre des motifs constitués d'au moins un monomère anionique préférablement choisi parmi ceux possédant les formules suivantes : 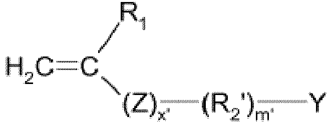 avec
Y est un groupe choisi parmi -COOH, -SO₃H, -OSO₃H,
- PO₃H₂ et -OPO₃H₂, les groupes SO₄H₂ et PO₄H₂ étant liés à R'₂ via l'atome d'oxygène, les groupes SO₃H et PO₃H étant liés à R'₂, respectivement, via les atomes S et P,
- R₁ représente un atome d'hydrogène ou un groupe alkyle contenant de 1 à 3 atome(s) de carbone, préférablement méthyle,
- Z est un groupe divalent choisi parmi -COO-, - CONH-, -CONCH₃-, -OCO-, et -O-, -SO₂-, -CO-O-CO- et -CO-CH₂-CO-, préférablement Z est choisi parmi COO et CONH,
- x' = 0 ou 1, préférablement 1,
- R'₂ est un radical divalent à base de carbone saturé ou insaturé, éventuellement aromatique, linéaire, ramifié ou cyclique comportant 1 à 30 atome(s) de carbone, qui peut comprendre 1 à 18 hétéroatome(s) choisi(s) parmi O, N, S, F, Si et P ; l'hétéroatome ou les hétéroatomes pouvant être intercalé(s) dans la chaîne dudit radical R'₂ ou en variante ledit radical R'₂ pouvant être substitué par un ou plusieurs groupes comprenant ceux-ci tels qu'hydroxyle ou amino.

9. Utilisation selon la revendication 8, dans laquelle le monomère anionique est choisi parmi l'anhydride maléique, l'acide acrylique, l'acide méthacrylique, l'acide crotonique, l'acide itaconique, l'acide fumarique, l'acide maléique, l'acrylate de 2-carboxyéthyle (CH₂=CH-C(O)-O-(CH₂)₂-COOH) ; l'acide styrènesulfonique, l'acide 2-acrylamido-2-méthylpropanesulfonique, l'acide vinylbenzoïque, l'acide vinylphosphorique et le (méth)acrylate de sulfopropyle, et des sels correspondants.

10. Utilisation selon l'une quelconque des revendications 1 à 9, dans laquelle le polymère floculant comprend également des motifs constitués d'au moins un monomère non ionique choisis notamment parmi ceux possédant la formule suivante, seule ou en tant que mélange : 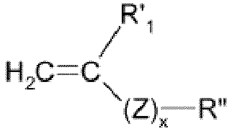 dans laquelle :
- R'₁ est hydrogène ou -CH₃ ;
- Z est un groupe divalent choisi parmi -COO-, - CONH-, -CONCH₃-, -OCO-, -SO₂-, -CO-O-CO- et -CO-CH₂-CO- et -0- ; préférablement COO et CONH ;
- x est 0 ou 1 ;
- R'' est un radical à base de carbone, saturé ou insaturé, éventuellement aromatique, linéaire, ramifié ou cyclique comportant 1 à 30 atome(s) de carbone, qui peut comprendre 1 à 18 hétéroatome(s) choisi(s) parmi O, N, S, F, Si et P ; l'hétéroatome ou les hétéroatomes pouvant être intercalé(s) dans la chaîne dudit radical ou en variante ledit radical R'' pouvant être substitué par un ou plusieurs groupes comprenant ceux-ci tels qu'hydroxyle, ester, amide, uréthane ou urée.

11. Utilisation selon la revendication 10, dans laquelle le monomère non ionique est choisi parmi le méthacrylate de méthyle, l'acrylate de méthyle, le méthacrylate d'éthyle, l'acrylate d'éthyle, l'acrylate de propyle, l'acrylate d'isopropyle, les méthacrylates de tétrahydrofurfuryle, le méthacrylate de butyle, l'acrylate de 2-éthylhexyle, le méthacrylate de stéaryle, l'acroléine, l'acrylate de tétrahydrofurfuryle, le méthacrylate de 2-hydroxyéthyle, l'acrylate de 2-hydroxyéthyle, le méthacrylate d'éthoxyéthyle, l'acrylate d'éthoxyéthyle, le N-isopropylacrylamide, le N-isopropylméthacrylamide, le N,N-diméthylacrylamide, le N,N-diméthylméthacrylamide, l'acétate de vinyle, le méthylvinyléther, l'éthylvinyléther, la vinylpyrrolidone, le vinylcaprolactame, le N-vinylacétamide, l'acrylate d'hydroxypropyle, le N-vinyllactame, l'acrylamide, le N-méthylacrylamide, le N,N-diméthylacrylamide, le N-méthyl-N-vinylacétamide, le N-vinylformamide, le N-méthyl-N-vinylformamide, l'alcool vinylique (copolymérisé sous forme d'acétate de vinyle et ensuite hydrolysé).

12. Utilisation selon l'une quelconque des revendications 1 à 11, dans laquelle le polymère floculant est choisi parmi :
- des copolymères poly(4- ou 2-vinylpyridine)-co-styrène ;
- des copolymères poly(4- ou 2-vinylpyridine)-co-méthacrylate de butyle ;
- poly(4- ou 2-vinylpyridine) réticulée avec le divinylbenzène ;
- des polymères diblocs comprenant un bloc polyéthylèneglycol (PEG) ou polyisobutylène et un bloc poly(4- ou 2-vinylpyridine) ;
- p-toluènesulfonate de poly(4-vinylpyridinium) ;
- 2% de poly(chlorhydrate de 4-vinylpyridine) réticulé avec le divinylbenzène.

13. Procédé cosmétique pour le traitement de la transpiration, qui consiste en l'application à la surface de la peau d'une composition comprenant, dans un milieu acceptable sur le plan cosmétique, au moins un polymère floculant tel que défini selon l'une quelconque des revendications 1 à 12, ne contenant aucun sels d'aluminium et/ou de zirconium antitranspirants.
